# EUROPEAN PATENT APPLICATION

(11) **EP 4 667 913 A1**
(43) Date of publication of application: **24.12.2025**
(21) Application number: 25183387.7
(22) Date of filing: 17.06.2025
(51) Int. Cl.: G01N 21/78, A61M 5/00, G01N 21/77

(54) **MEDICAMENT DELIVERY DEVICES WITH A SENSOR ASSEMBLY FOR DETERMINING ANALYTE CONCENTRATIONS**

(30) Priority: 20.06.2024 US 202463662178 P; 16.05.2025 US 202519210763
(71) Applicant: Medtronic MiniMed, Inc., Northridge, CA 91325 (US)
(72) Inventor: Romo-Anselmo, Evan M., Northridge (US); Zhang, Guanping, Northridge, 91325 (US); Patel, Ami V., Northridge, 91325 (US); Kwa, Timothy C., Northridge, 91325 (US); Chattaraj, Sarnath, Northridge, 91325 (US); Tong, Davy T., Northridge, 91325 (US)
(74) Representative: Zimmermann & Partner Patentanwälte mbB

(57) **Abstract**

Systems and methods for determining medicament concentrations within medicament delivery devices are disclosed. A medicament delivery device can include a fluid path comprising a reservoir in fluid communication with an outlet port, in which the device is actuatable to drive fluid through the fluid path, from the reservoir and out of the outlet port. A sample receiving chamber of the delivery device is configured to receive a fluid sample from the fluid path. The sample receiving chamber includes an analytical reagent configured to react with the fluid sample to modify the color of the fluid sample. A sensor assembly of the delivery device includes a detector configured to determine the color of the reacted fluid sample and one or more signal processing components configured to provide an indication of the analyte concentration in the fluid sample based at least in part on the color determination.

## Description

### CROSS-REFERENCE TO RELATED APPLICATIONS

This application claims the benefit of priority to U.S. Provisional Application No. 63/662,178, filed June 20, 2024, which is incorporated herein by reference in its entirety.

### TECHNICAL FIELD

The present technology relates generally to medical devices, and more particularly, to systems and methods for sensing analyte concentrations and/or compositions for medicament delivery devices.

### BACKGROUND

Insulin delivery devices, such as insulin pumps, have become increasingly popular for managing diabetes by providing a convenient and accurate means of administering insulin to patients. These devices enable users to adjust insulin delivery based on their individual needs and requirements. If a certain insulin delivery device is designed to accommodate different concentrations of insulin, this presents a potential risk to users. For instance, a given tethered and/or patch pump may be labeled to be compatible with multiple insulin concentrations of, for example, 100 units per milliliter (U100), 200 units per milliliter (U200), 500 units per milliliter (U500), and even up to 1000 units per milliliter (U1000).

One significant challenge associated with insulin delivery devices that can accommodate various insulin concentrations is the possibility of users failing to properly adjust the pump settings for a given insulin concentration. This oversight can lead to either an over-dosing or under-dosing of insulin dispensed by the pump, which may result in serious health consequences for the user. Incorrectly adjusted pump settings can cause hypoglycemia or hyperglycemia, both of which can have detrimental effects on a patient's well-being.

Therefore, there is a need for a system that can accurately determine the concentration of insulin for an insulin delivery device and ensure that the device's settings are properly adjusted to match the detected insulin concentration. Such a system can help mitigate the risks associated with user error and improve the overall safety and efficacy of insulin delivery devices.

### SUMMARY

Generally, in some embodiments in accordance with the present technology, a sample analyzer includes a sample receiving region having an analytical reagent. The sample analyzer is configured to receive a fluid sample of a medicament at the sample receiving region. The analytical reagent is configured to react with the fluid sample to modify a color of the fluid sample. The color of the reacted fluid sample can be determined, and the sample analyzer can provide an indication of the analyte concentration in the fluid sample. Accordingly, the sample analyzer can be utilized in medicament verification and adjustment and a variety of other medical applications. The present technology is additionally or alternatively illustrated, for example, according to various aspects described below. These are provided as examples and do not limit the subject technology.

Generally, in some embodiments in accordance with the present technology, a medicament delivery device includes a fluid path which includes a reservoir in fluid communication with an outlet port. The device is actuatable to drive fluid through the fluid path, from the reservoir and out of the outlet port. The device further includes a sample receiving chamber coupled with the fluid path, the sample receiving chamber configured to receive a fluid sample from the fluid path. The sample receiving chamber includes an analytical reagent configured to react with the fluid sample to modify a color of the fluid sample. The device additionally includes a sensor assembly with a detector configured to determine the color of the reacted fluid sample and one or more signal processing components configured to provide an indication of the analyte concentration in the fluid sample based at least in part on the color determination.

In some aspects, the analyte comprises insulin. The analytical reagent can be a chelating agent (e.g., dithizone). Optionally, the analytical reagent is configured to react with the fluid sample to form one or more coordination complexes, and the one or more coordination complexes provides the modified color of the fluid sample. The detector can be configured to determine the color of the reacted fluid sample by determining a dominant wavelength of the one or more coordination complexes.

In some aspects, the sample receiving chamber is coupled with the fluid path via a one-way valve to prevent fluid flow from the sample receiving chamber to the fluid path. The sample receiving chamber can be coupled to the reservoir, to the outlet port, or to another location. The sample receiving chamber can be releasably coupled with the fluid path. In some aspects, the sample receiving chamber comprises one or more optical indicators, and the indication of the analyte concentration is provided via the one or more optical indicators.

In some aspects, the signal processing components are further configured to, based at least in part on the indication of the analyte concentration in the fluid sample, cause additional actions to be taken. The additional actions can include: causing an alarm to be output to a user, causing a dispensation mechanism to be adjusted, wherein the adjustment varies an amount or rate of fluid dispensed via the medicament delivery device, and/or inhibiting dispensation of fluid via the medicament delivery device.

Generally, in some embodiments in accordance with the present technology, a colorimetric analysis device includes a housing having a window and a sample receiving region disposed in the housing, the sample receiving region comprising an analytical reagent. The sample receiving region is configured to receive a fluid sample of a medicament and modify a color of the fluid sample by reacting the fluid sample with the analytical reagent. The color-modified fluid sample is visible through the window, and the modified color is indicative of the analyte concentration in the fluid sample.

In some aspects, the analytical reagent is configured to react with the fluid sample to form one or more coordination complexes, and the color-modified fluid sample comprises the one or more coordination complexes. The modified color can be identified by determining a dominant wavelength of the one or more coordination complexes. Optionally, the analyte concentration in the fluid sample can be determined from an image captured by a camera. In some examples, the image is processed by one or more image processing components to determine the analyte concentration.

In some aspects, the sample receiving region comprises a plurality of capillaries, and wherein the capillaries are configured to independently process the fluid sample. The device can further include a reference sample for calibration, wherein the reference sample contains fluid having a known color. The analyte can comprise insulin. The analytical reagent can comprise a chelating agent (e.g., dithizone).

Generally, in some embodiments in accordance with the present technology, a method includes disposing a fluid sample comprising medicament within a sample receiving region, wherein the sample receiving region comprises an analytical reagent. The method further includes reacting the fluid sample with the analytical reagent to modify a color of the fluid sample, determining the color of the reacted fluid sample, and, based on the determined color, providing an indication of an analyte concentration in the fluid sample.

In some aspects, the analyte comprises insulin. The analytical reagent can comprise a chelating agent (e.g., dithizone). Optionally, reacting the fluid sample with the analytical reagent comprises forming one or more coordination complexes, and wherein the one or more coordination complexes produces the modified color.

In some aspects, the sample receiving region is enclosed within a medicament delivery device configured to deliver the medicament to a patient. Additionally or alternatively, the sample receiving region can be isolated from the rest of the medicament delivery device via a one-way valve. In some examples, the method further comprises, based at least in part on the indication of the analyte concentration in the fluid sample, causing a dispensation mechanism to be adjusted, wherein the adjustment varies an amount or rate of fluid dispensed via a medicament delivery device. In some aspects, the method further comprises, based at least in part on the indication of the analyte concentration in the fluid sample, inhibiting dispensation of fluid via a medicament delivery device. The determination can be based on an image captured by a camera. Optionally, the determination is made using an optical detector comprising an emitter and a receiver.

Further disclosed therein are systems and methods for determining medicament concentrations within medicament delivery devices. A medicament delivery device can include a fluid path comprising a reservoir in fluid communication with an outlet port, in which the device is actuatable to drive fluid through the fluid path, from the reservoir and out of the outlet port. A sample receiving chamber of the delivery device is configured to receive a fluid sample from the fluid path. The sample receiving chamber includes an analytical reagent configured to react with the fluid sample to modify the color of the fluid sample. A sensor assembly of the delivery device includes a detector configured to determine the color of the reacted fluid sample and one or more signal processing components configured to provide an indication of the analyte concentration in the fluid sample based at least in part on the color determination.

Other technical features may be readily apparent to one skilled in the art from the following figures, descriptions, and claims.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. 1 is a schematic block diagram of a sample analyzer in accordance with several embodiments of the present technology.
FIG. 2 is a graph of hue values over time for different fluid samples having varying concentrations of insulin.
FIG. 3 is a partially exploded view of a sample analyzer enclosed in a housing in accordance with several embodiments of the present technology.
FIG. 4 is a schematic perspective view of an example medicament delivery assembly including a sample analyzer in accordance with several embodiments of the present technology.
FIG. 5A is a schematic perspective view of an example medicament delivery assembly including a sample analyzer in accordance with several embodiments of the present technology.
FIG. 5B is an enlarged perspective view of a portion of the medicament delivery assembly shown in FIG. 5A.

### DETAILED DESCRIPTION

### I. Overview

The present technology relates to systems and methods for determining the concentration of a medicament for a fluid delivery device, such as for detecting insulin concentration for use with an insulin pump or other suitable insulin delivery device. In some implementations, a sample analyzer system utilizes colorimetric approaches to detect insulin concentration, providing a convenient and accurate means of monitoring and/or adjusting insulin delivery.

In one aspect, a fluid delivery device includes various fluid delivery components, such as a reservoir, an outlet port, and a fluid conduit extending therebetween, with at least one sample receiving chamber for analysis of a fluid sample. In some examples, the fluid can include an analyte, such as insulin or other medicament, as well as one or more diluents. The sample receiving chamber can include an analytical reagent configured to react with a fluid sample to modify the color of the fluid sample. A detector is configured to determine the color of the reacted fluid sample and signal processing component(s) are configured to provide an indication of the analyte concentration of the analyte, such as insulin or other medicament, based at least in part on the color determination.

In another aspect, a colorimetric analysis device is configured to receive a fluid sample, for example, from a fluid delivery device similar to the one described above. The colorimetric analysis device includes a housing having a window and a sample receiving region disposed in the housing. In some examples, the fluid sample can include an analyte, such as insulin or other medicament, as well as one or more diluents. The sample receiving region can include an analytical reagent configured to react with the fluid sample to modify the color of the fluid sample. The reacted sample, also referred to herein as a color-modified fluid sample, may be visible through the housing window, and the analyte concentration of the analyte, such as insulin or other medicament, can be determined by analyzing the reacted sample.

The present technology offers several advantages, for instance providing the ability to accurately determine insulin concentration in a non-contact, non-invasive manner, enabling further miniaturization of insulin delivery devices and longer wear times for patients by facilitating the use of higher-concentration insulin. By ensuring proper adjustment of device settings based on detected insulin concentration, the technology improves the safety and efficacy of insulin delivery, reducing the risk of over- or under-dosing due to user error. Additionally, several embodiments of the technology provide for a portable, easy-to-use, and self-administrable test for verifying insulin concentration, improving patient compliance and confidence in treatment.

### II. Example Optical Sensor Assemblies for Analyte Concentration Determinations

### A. Sample Analyzer Overview

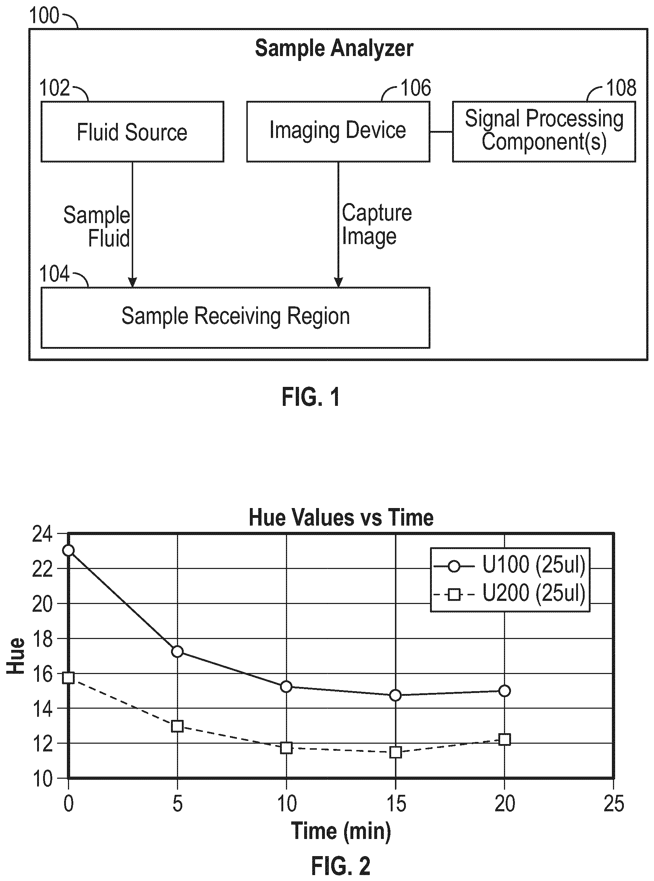

Figure 1 illustrates a schematic block diagram of a sample analyzer 100 according to some embodiments of the present technology. The sample analyzer 100 comprises a fluid source 102 configured to dispose a fluid sample into a sample receiving region 104. The sample receiving region 104 can be configured to react with the fluid sample to modify a color of the fluid sample. Imaging device 106 can be a detector configured to capture an image of the sample receiving region 104. The image can be processed by one or more signal processing components 108 to determine the color of the reacted sample in the sample receiving region 104. The sample analyzer 100 can provide an indication of the analyte concentration in the fluid sample based at least in part on the determined color of the reacted sample.

In some embodiments, the fluid source 102 can be coupled with the sample receiving region 104, for instance being integrated into a single device together. In some examples, the fluid source 102 and the sample analyzer 100 can be part of a medicament delivery device configured to deliver medicament to a patient. The fluid source 102 can include an analyte, such as insulin or other medicament, and one or more diluents. In some embodiments, the concentration of the analyte in the fluid source 102 is initially unknown. For instance, the fluid source 102 may include an insulin formulation of an unknown insulin concentration. The sample analyzer 100 can be configured to differentiate between different insulin formulations (e.g., between U100, U200, and U500 formulations).

While the fluid source 102 can form a part of the sample analyzer 100, in some embodiments, the fluid source 102 is separate from the sample analyzer 100. For instance, the fluid source 102 can be part of a medicament delivery device for delivering medicament to a patient, or can be a standalone fluid source (e.g., a syringe having a barrel storing the fluid and a needle configured to emit fluid from the barrel). Accordingly, the sample receiving region 104 can be configured to receive a fluid sample from the fluid source 102 via extraction (e.g., pipetting and/or through a nozzle, sealable valve, etc.). In some embodiments, the fluid sample is withdrawn from the fluid source 102 by a patient or medical practitioner and disposed in the sample receiving region 104. For instance, the fluid sample may be withdrawn and analyzed during a priming stage prior to medicament administration.

The sample receiving region 104 can be configured to process, interact with, modify, and/or transform the fluid sample, producing the reacted sample. In some embodiments, the sample receiving region 104 is configured to modify a color of the fluid sample, e.g., through a chemical process. The sample receiving region 104 may comprise an analytical reagent having a sufficient reactivity with the fluid sample. In some examples, the analytical reagent can be configured to undergo an immediate reaction with the fluid sample. Alternatively, the analytical reagent can be configured to undergo a delayed and/or prolonged reaction with the fluid sample. Various additional components may be present in the sample receiving region 104 such as catalysts and/or additional reactants that can affect the fluid sample.

The sample receiving region 104 can be observed by the imaging device 106. The imaging device 106 can be portable, hand-held, or stationary, and can take the form of a separate device that is not connected to the sample receiving region. Suitable imaging devices for capturing images of the sample receiving region 104 include a smartphone, tablet, digital camera, etc. Additionally or alternatively, the imaging device 106 can be integrated into a common device with the sample receiving region 104, for instance with both the imaging device 106 and the sample receiving region 104 being integrated into a common medicament delivery device as described in more detail below. In some embodiments, the imaging device 106 automatically captures images of the sample receiving region 104. Alternatively, or in combination, the imaging device 106 can be prompted, for example, by a user or medical practitioner, to capture an image of the sample receiving region 104.

The imaging device 106 can be operatively coupled with the signal processing components 108 for analyzing the captured image(s). The signal processing components 108 can also instruct and assist a user in capturing images. As described in more detail below, the signal processing components 108 can be configured to determine the analyte concentration in the fluid sample based on the color of the reacted sample. As used herein, "capturing an image" of the reacted sample can include any form of directly or indirectly detecting photons received from the sample receiving region 104 and storing signals based on the detected photons, whether or not this detection results in construction of a graphical representation of the detected photons.

In some embodiments, the imaging device 106 comprises an optical emitter and an optical detector. The optical emitter can be configured to emit photons as an input beam towards the reacted sample. After the input beam interacts with the reacted sample, an output beam is generated, which comprises photons that have passed through and/or are reflected from the reacted sample. The optical detector can be configured to receive the photons of the output beam and provide a detector signal to the signal processing components 108. In turn, the one or more signal processing components 108 can be configured to receive the detector signal from the optical detector and, based at least in part on the detector signal, provide an indication of the analyte concentration in the fluid sample.

In some embodiments, the optical detector is configured to detect photons passing through the reacted sample, and the signal processing components 108 are configured to determine the analyte (e.g., medicament) concentration based on evaluating the photons (e.g., by assessing the color or hue of the photons).

The sample analyzer 100 may further comprise additional component(s), which may include communication components (e.g., wireless transceivers), drive mechanisms for adjusting dispensation of fluid via a medicament delivery device comprising the fluid source 102, and any other suitable components of a sample analyzer 100.

As further detailed herein, the sample analyzer 100 can be configured to determine the analyte concentration in the fluid sample based on the color of the fluid sample after the fluid sample undergoes a chemical reaction. In some embodiments, the determination is based on identifying the hue of the reacted sample. This can be performed, for example, by determining the dominant wavelength of the reacted sample in a captured image from the imaging device 106 using the signal processing components 108. Alternatively, or in combination, the determination can be based on one or more of the reacted sample's saturation, shade, tone, intensity, tint, chroma, or brightness. In some embodiments, the determination can be made by comparing the color of the reacted sample with known medicament-associated colors. For instance, the sample analyzer 100 may comprise one or more color tags, wherein each of the color tags corresponds to an analyte concentration. This can enable a user or medical practitioner to quickly reference the color tags and determine the analyte concentration by comparing the reacted sample with the color tags.

In some embodiments, the sample analyzer 100 and/or external devices for use with the sample analyzer 100 comprise a memory configured to store known associations between analyte concentration and color. The memory may have stored experimental data and/or dictionaries for differentiating between analyte concentrations based on color. Such mappings are now illustrated with respect to an example colorimetric analysis.

Figure 2 presents an example graph depicting the results of a colorimetric analysis of two insulin formulas that have reacted with dithizone. As described in more detail below, when dithizone reacts with the zinc ion bound to insulin (which is present in standard insulin formulations), the resulting complex has a different hue than that of either the dithizone or the insulin alone. The graph compares the difference in hue values over reaction time between U100 insulin (100 units of insulin per milliliter of fluid) and U200 insulin (200 units of insulin per milliliter of fluid). As shown, the U200 insulin has a lower hue value than the U100 insulin at all timepoints. Hue values range from 0 to 360, corresponding to an angular position in color space. When insulin reacts with a suitable analytical reagent (e.g., dithizone or other suitable reagent as described herein), the insulin and the analytical reagent can form one or more coordination complexes comprising a specific hue. Changing the concentration of the insulin can affect the resultant hue of the one or more coordination complexes and thus the concentration of insulin can be derived from the resultant hue. For instance, at 5 minutes, the U200 insulin has a hue value around 13 and the U100 insulin has a hue value around 17. Given an unknown insulin formula, after a 5-minute test, a sample analyzer can determine a hue value of 13 and accordingly identify that the unknown insulin formula is U200 insulin. Similar approaches can be taken for mapping other color properties such as saturation and brightness to analyte concentration. Optionally, the colorimetric analysis can include normalization processes based on known reference samples. For instance, the sample analyzer may be supplied with a known U100 insulin sample and the fluid sample can be compared to the known U100 insulin sample.

### C. Example Sample Receiving Regions

As noted above, the sample receiving region 104 is responsible for reacting the fluid sample with one or more reagents such that the analyte concentration of the fluid sample can be determined. While various reagents of the sample receiving region 104 are described herein, it will be appreciated that the sample receiving region 104 can be adapted to include various other chemical compounds and the sample receiving region 104 can be configured to provide for a plurality of chemical processes simultaneously or in series.

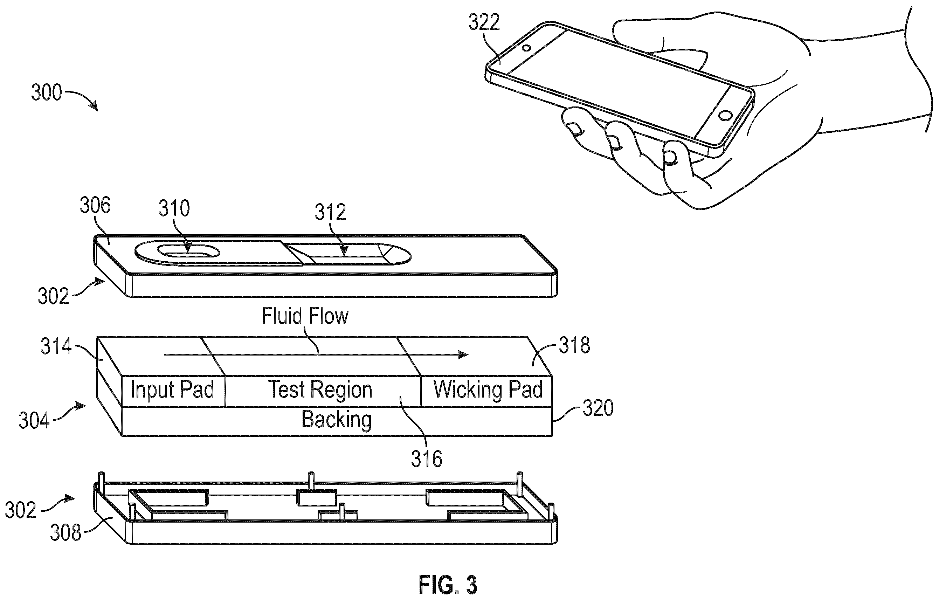

Furthermore, the sample receiving region 104 may alternatively or additionally include physical and/or mechanical features to assist in the reaction. For instance, the sample receiving region 104 may include one or more gradients to assist in the flow of the fluid sample across the sample receiving region 104. In some embodiments, the sample receiving region 104 includes a plurality of capillaries and/or channels for simultaneous and/or independent assessment of the fluid sample. Alternatively, the sample receiving region 104 can include a drip chamber and/or funnel for transferring fluid. The sample receiving region 104 may be partitioned into a plurality of functional regions. For instance, the sample receiving region 104 can comprise a multi-region assay, as discussed below with respect to FIG. 3.

In some examples, the sample receiving region 104 includes an analytical reagent. The sample receiving region 104 can be coated with the analytical reagent. Alternatively, or in combination, the analytical reagent can be disposed on the sample receiving region 104.

The analytical reagent can be a chelating agent. Chelating agents, also known as sequestering agents, bind tightly to metal ions, forming new chemical compounds. Some chelating agents are highly specific for a target metal ion, such as iron, copper, mercury or lead. Other chelating agents can be used with multiple types of metal ions. Chelating agents suitable for use with the present technology include dithizone, zincon, cobalticyanide, and/or spirooxazines. Chelation can affect the color of the chemical compound. For instance, when dithizone binds to Zinc ions in insulin, the resultant coordinate complex comprises a pinkish hue. For different insulin concentrations, the color (e.g., hue) may change when reacted with dithizone. As used herein, changes in color can refer to changes in hue (e.g., the dominant wavelength, spectrum of wavelengths, etc.), in intensity or brightness, any other color parameter, or any combination thereof.

The sample receiving region 104 can include porous and/or absorbent materials. In some embodiments, the sample receiving region 104 is a continuous pad, test strip, or sheet. Optionally, the sample receiving region 104 can comprise paper. For example, the paper can be infused with the analytical reagent.

In some examples, the sample receiving region 104 further comprises a reference sample. The reference sample can be stored and/or disposed in the sample receiving region 104. In some embodiments, the reference sample is a fluid having a known color (e.g., red). In some embodiments, the reference sample may be utilized in a calibration process of the sample analyzer 100.

In some examples, the reference sample can be a medicament sample that is processed and analyzed similarly to the fluid sample. For instance, the reference sample can comprise a known insulin concentration, e.g., the reference sample can include U100 insulin, U200 insulin, U500 insulin, etc. The analytical reagent of the sample receiving region 104 can react with the reference sample to modify the color of the reference sample. Since the reference sample has a known insulin concentration, the color of the reacted reference sample can be compared to the expected color corresponding to the known insulin concentration. Various operations of the sample analyzer 100 can be modified based on the reference sample, as will be discussed further herein.

### D. Example Imaging Devices

In some embodiments, the imaging device 106 is part of the sample analyzer 100. In other embodiments, the imaging device 106 is a separate device from the sample analyzer 100. The imaging device 106 can include storage, processing, and/or communication circuitry. The imaging device 106 can be operatively linked with signal processing components 108. For instance, the imaging device 106 can include one or more wired or wireless emitters and receivers for communicating with the signal processing components 108. In some embodiments, the signal processing components 108 are housed within the imaging device 106.

The imaging device 106 is responsible for capturing color data of the reacted sample which can be used to determine the color of the reacted sample. In a first aspect, the imaging device 106 can be configured to capture an image of the sample receiving region 104. The imaging device 106 can comprise a charge coupled device (CCD), electron multiplying charge coupled device (EMCCD), complementary metal oxide semiconductor (CMOS), scientific complementary metal oxide semiconductor (sCMOS), or any other suitable image sensor. The imaging device 106 can be configured to capture light and convert the light into an electrical signal. The electrical signal can then be used to produce a digital image. In such cases, the digital image may be processed by the signal processing components 108 as discussed further herein. The imaging device 106 can be part of a smartphone, tablet, webcam, and/or digital camera.

In a second aspect, the imaging device 106 can be configured to convert light emitted from the reacted sample into an electrical signal that can be directly processed and analyzed to determine the color of the reacted sample. In such cases, the imaging device 106 can include an optical detector, and optionally, an optical emitter. The optical emitter can be configured to produce an input beam of energy towards the reacted sample, wherein the interaction between the input beam and the reacted sample produces an output beam. The optical emitter can include any suitable light source, such as an LED, laser, or other suitable component configured to illuminate the reacted sample. While various light sources are described herein, it will be appreciated that the optical emitter can also include auxiliary optical components, such as lenses, mirrors, gratings, filters, etc. so as to achieve a desired output beam of photons, in terms of intensity, wavelength, and direction.

The optical detector can detect photons from the output beam, e.g., as an electrical signal, and the sample analyzer 100 can determine the analyte concentration in the fluid sample based at least in part on the electrical signal. The optical detector can also detect photons emitted from the reacted sample directly.

Where the sample analyzer 100 comprises a reference sample, the imaging device 106 can additionally be configured to capture an image of a reacted reference sample. In some examples, the imaging device 106 can capture an image of both the reacted reference sample and the reacted sample simultaneously. However, it is also possible to capture an image of the reacted reference sample and then compare the image of the reacted reference sample with a later-captured image of the reacted sample.

### E. Example Signal Processing Components

Where the color data captured by the imaging device 106 comprises a captured image, the signal processing components 108 of the sample analyzer 100 can be configured to convert the captured image into a meaningful indication of the analyte concentration. Where the color data captured by the imaging device 106 comprises a raw signal, the signal processing components 108 of the sample analyzer 100 can be configured to convert the raw signal into a meaningful indication of the analyte concentration.

The signal processing components 108 can optionally include a variety of analog and/or digital circuitry, depending on the specific requirements and constraints of the application. In some embodiments, the signal processing components 108 are stored proximate to the sample receiving region 104 of the sample analyzer 100. Alternatively, or in combination, the signal processing components 108 can be stored in the imaging device 106.

An example of a signal processing component 108 is an image processor. The image processor can determine the dominant wavelength in the reacted sample based on a digital image of the reacted sample. The image processor can additionally or alternatively determine the brightness, saturation, chromaticity, shade, intensity, tint, tone, etc. of the reacted sample. In some embodiments, the signal processing component 108 converts the digital image into RGB (Red-Green-Blue) and/or HSB (hue-saturation-brightness) values, and the RGB and/or HSB values are used to determine the analyte concentration in the fluid sample. Alternatively, or in combination, the signal processing component 108 can apply filters or otherwise manipulate the data from the imaging device 106.

In some applications, it may be sufficient to sort readings of analyte concentration in the fluid sample into one of a predefined number of concentration ranges, rather than determining the exact concentration value. This can simplify the signal processing requirements and reduce the cost and complexity of the sample analyzer 100. For example, in the case of insulin, it may be desirable to distinguish between U100, U200, and U500 formulations, which have different concentrations of insulin per unit volume.

To achieve concentration range sorting, the signal processing components 108 can include a series of analog comparators or digital threshold detectors. These components compare the amplitude or other characteristics of the detector signal to predefined threshold values corresponding to the different concentration ranges. For example, if the detected hue falls within a predetermined range, it may indicate a U100 insulin formulation, while a signal falling within another predetermined range may indicate a U500 formulation. The output of the comparators or threshold detectors can be used to drive indicators (e.g., LEDs) or generate digital codes that identify the concentration range of the sample.

Another approach to concentration range sorting is to use pattern recognition techniques, such as principal component analysis (PCA) or linear discriminant analysis (LDA). These techniques can be applied to the digitized detector signal to extract features that are characteristic of the different concentration ranges, and then classify the fluid sample based on its similarity to the reference patterns. This can be implemented using software running on a microprocessor or FPGA, or using specialized hardware accelerators for machine learning.

In various implementations, data gathered from the imaging device 106 can be compared to reference data to determine resulting analyte concentration levels. Such data may be stored in lookup tables, such as pre-calculated arrays that map given input values (e.g., raw or processed signal data from the imaging device 106) to output values (e.g., analyte concentration or other parameter). In some examples, a mathematical model can be stored that represents the relationship between the incoming signals from the imaging device 106 and the resulting analyte concentration. Such mathematical models can include polynomial regression models, neural network models, classification models, or any other suitable techniques.

Where the sample analyzer 100 comprises a reference sample, the signal processing components 108 can be additionally configured to calibrate the sample analyzer 100 based on the reference sample. For instance, the signal processing components 108 can determine the color of a reacted reference sample. The determined color of the reacted reference sample can be compared to the expected (e.g., known) color of the reacted reference sample. Thereafter, the sample analyzer 100 can adjust future measurements and analysis based on the difference (or lack thereof) between the determined color of the reacted reference sample and the expected color. In some embodiments, for example, the determined color of a reacted sample is adjusted by the signal processing components 108 based on the calibration.

### F. Example Actions Responsive to Concentration Determinations

The integration of the sample analyzer 100 into a medicament delivery device enables the implementation of various automated actions based on the determined analyte concentration. For instance, based on a determined concentration of analyte (e.g., insulin), a medicament delivery device can perform one or more actions automatically. These actions can help ensure user safety, improve the efficacy of the treatment, and enhance the overall user experience. While the automated action can take any suitable form, three examples are described herein: (1) outputting an alarm to the user, (2) automatically adjusting the dispensing mechanism, and (3) inhibiting medicament dispensation.

Outputting an alarm to the user can serve as a safety feature that is triggered when the sample analyzer 100 detects an analyte concentration that differs significantly from the expected or programmed value. For example, if the user has loaded a U200 insulin cartridge into a device programmed for U100 insulin, the higher concentration could lead to an overdose if not detected and corrected. In this case, the signal processing components 108 can compare the measured concentration to the expected value and trigger an alarm if the difference exceeds a predetermined threshold (e.g., 20%).

The alarm can be implemented in various forms, such as an audible buzzer, a visual indicator (e.g., a flashing LED or a warning message on a display), or tactile feedback (e.g., a vibration). The alarm can also be accompanied by a message prompting the user to check the cartridge and reprogram the device if necessary. By alerting the user to potential concentration mismatches, the sample analyzer 100 can help prevent medication errors and improve patient safety. In another embodiment, the alarm can take the form of a confirmatory alarm. In such an embodiment, the alarm would require the user to confirm the detected concentration before the infusion device is enabled to provide therapy to the user. The confirmatory alarm is meant to be a double check before the user is dispensed any medication. For example, the alarm could display on a mobile device that U100 is detected and require the user to confirm that U100 is the insulin they intended to load into the device. Only after the user confirms the concentration will the device begin therapy delivery.

Automatically adjusting the dispensing mechanism is another feature enabled by the sample analyzer 100. In some cases, the user may intentionally load a cartridge with a different concentration than the one previously used, in order to adjust the dosage or accommodate changes in their treatment plan. In such situations, the sample analyzer 100 can detect the new concentration and automatically adjust the dispensing mechanism to maintain the desired dose. For example, if the user switches from a U100 insulin cartridge to a U200 cartridge, the sample analyzer 100 can detect the higher concentration and signal the dispensing mechanism to reduce the volume of fluid dispensed per unit time, in order to maintain the same effective dose of insulin. Conversely, if the user switches to a lower concentration cartridge, the dispensing mechanism can be adjusted to increase the volume of fluid dispensed per unit time. This automatic adjustment can be achieved by modifying the control parameters of the dispensing mechanism, such as the stroke volume of a piston pump or the duty cycle of a peristaltic pump.

Inhibiting medicament dispensation is a third safety feature that can be implemented based on the concentration determination by the sample analyzer 100. In some cases, the detected concentration may be so far outside the expected range that it indicates a potential hazard to the user, such as a contaminated or degraded medicament. In such situations, the signal processing components can trigger a complete shutdown of the dispensing mechanism to prevent any further delivery of the suspect medicament.

The inhibition of medicament dispensation can be implemented by disabling the power supply to the dispensing mechanism, closing a safety valve in the fluid path, or engaging a mechanical lock to prevent actuation of the mechanism. The inhibition can be accompanied by an alarm and a message to the user, indicating the reason for the shutdown and advising them to replace the cartridge or seek medical attention if necessary. By preventing the delivery of potentially harmful medicaments, the sample analyzer 100 can provide an additional layer of protection for the user.

### III. Example Sample Analyzers for Colorimetric Concentration Detection

Figure 3 illustrates an example colorimetric analysis device 300 configured to determine an analyte concentration in a fluid sample. In various implementations, the colorimetric analysis device 300 can be a single-use, disposable assay that enables a user (e.g., a patient or medical practitioner) to provide a fluid sample containing medicament (e.g., liquid insulin) and determine the concentration based on colorimetric analysis. The colorimetric analysis device 300 can be configured to process the fluid sample using one or more chemical and/or physical processes. In some embodiments, the colorimetric analysis device 300 is portable and light. For instance, the colorimetric analysis device 300 can be carried around by a user and utilized to verify the appropriate medicament before the medicament is self-administered by the user, e.g., via a medicament delivery device. The colorimetric analysis device 300 can generally include a housing 302 and a sample receiving region 304 disposed in the housing 302, the sample receiving region 304 configured to receive a fluid sample of a medicament.

The housing 302 can include a cover 306 and base 308. In some embodiments, the cover 306 is detachable from the base 308, enabling the user to access and/or replace the sample receiving region 304. The housing 302 can further comprise a port 310 through which a fluid sample can be directed. The port 310 can be in fluid communication with the sample receiving region 304 via one or more capillaries and/or channels. Alternatively, the sample receiving region 304 may be directly connected to the port 310, e.g., the sample receiving region 304 is proximate to the port 310. In some embodiments, the cover 306 can include a plurality of ports 310 for independently receiving and assessing a plurality of fluid samples.

In some embodiments, the cover 306 comprises a window 312. The window 312 can be an open window exposing the sample receiving region 304. Optionally, the window 312 can include a thin transparent film. Furthermore, the cover 306 can comprise additional windows 312. For instance, where the sample receiving region 304 is configured to receive a plurality of fluid samples, the additional windows can correspond to each of the plurality of fluid samples.

The sample receiving region 304 can comprise an input pad 314, a test region 316, a wicking pad 318, and a backing 320. The sample receiving region 304 may receive the fluid sample at the input pad 314 (e.g., after the fluid sample is delivered through the port 310). From the input pad 314, the fluid sample may be drawn toward the test region 316 and the wicking pad 318. The sample receiving region 304 can include one or more multi-region capillaries, and the fluid may be drawn from the input pad 314 toward the test region 316 and the wicking pad 318 due at least in part to capillary forces. In some embodiments, the input pad 314 is configured to subdue the downstream release of the fluid sample. For instance, the input pad 314 can be configured to reserve a volume of the fluid sample within the input pad 314 until the volume of the fluid sample exceeds the capacity of the input pad 314. Alternatively, or in combination, the input pad 314 can comprise one or more chemical filters for preventing the flow of undesired chemical compounds into the test region 316.

The test region 316 can be configured to react with the fluid sample to modify the color of the fluid sample, producing a reacted sample. In some embodiments, the test region 316 includes an analytical reagent. Analytical reagents are commonly used in wet chemistry, chromatography, spectroscopy, water analysis, electrochemistry, and more. The test region 316 can be coated with the analytical reagent. Other suitable methods for binding the analytical reagent to the test region 316 can be applied. The analytical reagent can be evenly distributed across the test region 316. Alternatively, the analytical reagent may be concentrated in predetermined zones of the test region 316. For instance, the analytical reagent may be highly concentrated near the input pad 314 and sparse near the wicking pad 318.

The analytical reagent can comprise one or more chemical agents for reacting with the fluid sample. For instance, the analytical reagent can include a chelating agent, such as dithizone. Chelating agents bind to metal ions, making them useful in reacting with medicaments having metal ions. In embodiments where the chelating agent is dithizone, the dithizone can react with zinc ions found in insulin formulations (e.g., insulin hexamers). The analytical reagent can react with the fluid sample to modify the color of the fluid sample. When dithizone binds to the zinc, the resulting complex has a modified color, specifically having a pinkish hue.

The wicking pad 318 can be configured to absorb any excess reagents not bound to the fluid sample. The wicking pad 318 can also be configured to prevent the backflow of the fluid sample to the test region 316 and/or the input pad 314. In some embodiments, the wicking pad 318 includes a cellulose filter. The wicking pad 318 can comprise any suitable absorbent material for preventing the buildup of excess fluid in the sample receiving region 304.

The input pad 314, test region 316, and wicking pad 318 can be disposed on the backing 320. The backing 320 can be an inert elongate member. In some embodiments, the input pad 314, test region 316, and wicking pad 318 are coupled to the backing 320 via one or more of adhesives, frictional forces, gravitational forces, etc. The backing 320 can have a height within the range of 0.1 mm to 10 mm. In some embodiments, the backing 320 is adhered to the base 308. Alternatively, or in combination, the backing 320 can be secured in the housing 302 using one or more interlocking members or protrusions of the housing 302.

After the analytical reagent of the sample receiving region 304 has reacted with the fluid sample, an image of the sample receiving region 304 can be captured by an imaging device 322. The imaging device 322 can be the same or substantially similar to any of the imaging devices described herein, such as the imaging device 106 of the sample analyzer 100 of FIG. 1. In some embodiments, the imaging device 322 is a smartphone, tablet, or other portable computing device. The imaging device 322 can be configured to capture an image of the sample receiving region 304, through the window 312, and determine the color of the reacted sample. Based at least in part on the color of the reacted sample, the analyte concentration in the fluid sample can be determined. For instance, the imaging device 322 can be operatively linked with signal processing components configured to determine the analyte concentration.

In some implementations, the computing device that includes the imaging device 322 (e.g., a smartphone, tablet, etc.) can be in communication with a medicament delivery device such as a wearable infusion pump. Based on a determination of the analyte concentration, the computing device may exchange data with the medicament delivery device, for instance to communicate the determined analyte concentration, issue alerts, warnings, alarms, instructions to disable medicament dispensation, or to take other suitable action.

### IV. Example Medicament Delivery Devices Incorporating Optical Sensor Assemblies

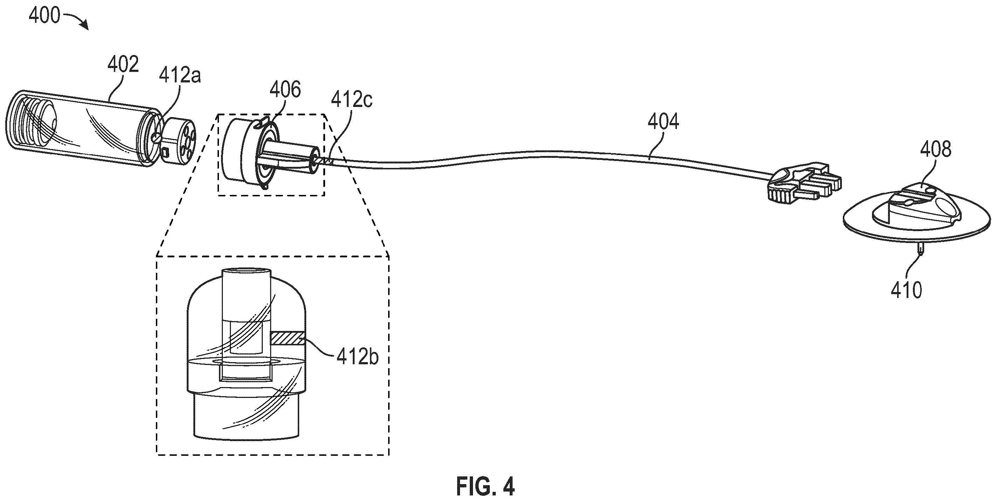

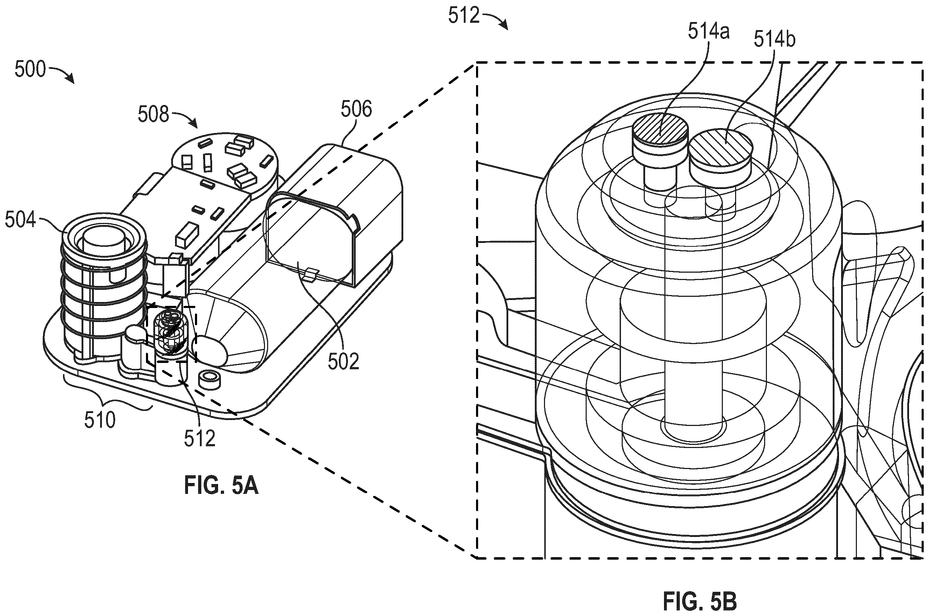

Figures 4, 5A, and 5B illustrate various example medicament delivery devices into which the sample analyzer 100, as described above, can be integrated. These devices represent different form factors and configurations of medicament delivery devices, each with its own unique features and advantages. However, it should be noted that these are only examples, and the sample analyzer 100 can be incorporated into a wide range of other medicament delivery devices, depending on the specific requirements and preferences of the patient and healthcare provider.

The integration of the sample analyzer 100 into these devices enables real-time, non-invasive monitoring of the analyte (e.g., medicament) concentration, which can significantly improve the safety, efficacy, and user experience of the medicament delivery process. By continuously measuring the analyte concentration at one or more points within the device's fluid path, the sample analyzer 100 can detect any deviations from the expected concentration and alert the user or healthcare provider to potential issues.

Furthermore, the incorporation of the sample analyzer 100 into these devices can facilitate the development of closed-loop control systems, where the medicament delivery is automatically adjusted based on the measured analyte concentration and other physiological parameters of the patient. This can lead to more personalized and optimized therapy, ultimately improving the patient's quality of life and treatment outcomes.

### A. Tethered Pump

Figure 4 illustrates an example medicament delivery device in the form of an infusion set 400 typically used with a tethered pump, which can incorporate the sample analyzer 100 described elsewhere herein. Figure 4 illustrates a reservoir 402 which can be placed in fluid communication with an infusion set 400 via an intervening conduit 404. The conduit 404 fluidly connects a cap 406 to the cannula housing 408. In operation, the reservoir 402 is housed within a pump (not shown), which can be worn by a user and actuated to drive fluid from the reservoir 402, through the cap 406 and along the conduit 404 to the cannula housing 408.

The reservoir 402 is responsible for storing and dispensing the medicament, such as insulin, to the patient. The reservoir 402 is typically coupled to a pumping mechanism for controlling the flow of the medicament, and various electronic components for regulating the pump's operation. The sample analyzer 100 can be integrated into the pump, for example, within the reservoir 402, to monitor the concentration of an analyte within the fluid being dispensed.

The cannula housing 408 is a device that is attached to the patient's body, usually via an adhesive patch, and delivers the medicament from the reservoir 402 into the patient's subcutaneous tissue. The cannula housing 408 typically includes a cannula 410 or needle that is inserted into the patient's skin, as well as a fluid path that connects the cannula 410 or needle to the conduit 404. The sample analyzer 100 can also be incorporated into the infusion set 400, anywhere within the fluid path, to measure an analyte concentration just before the fluid is delivered to the patient, such as in the cap 406 or in the cannula housing 408.

The conduit 404 is a flexible tube that carries the medicament from the reservoir 402 through the cap 406 to the cannula housing 408. It is typically made of a biocompatible material that is resistant to kinking and can withstand the pressure generated by the pump. The sample analyzer 100 can be integrated along the length of the conduit 404 at one or more locations to monitor an analyte concentration as the fluid travels from the reservoir 402 to the cannula housing 408.

The medicament delivery device can comprise one or more indicators 412a, 412b, and/or 412c ("indicators 412") configured to indicate the analyte concentration of the fluid. The indicators 412 can be positioned separately from the sample analyzer 100 or may be integral to the sample analyzer 100. Each of the indicators 412, 412b, and 412c can include a sample receiving region configured to receive a portion of the fluid therein. As noted above, the sample receiving region can include an analytical reagent (e.g., dithizone) to facilitate colorimetric analysis of the analyte concentration in the fluid. To avoid contamination of fluid to be delivered to the patient with the analytical reagent, a sample of the fluid can be diverted to the sample receiving region in a one-way, irreversible manner. For instance, a check valve, rotatable valve, duck-bill valve, or other suitable controllable or passive one-way valve can permit a small volume of fluid sample to pass into the sample receiving region that include the analytical reagent, while not permitting the received fluid sample to exit the sample receiving region. In such configurations, the sample analyzer 100 can be a single-use component. In some implementations, a plurality of separate sample receiving regions can be provided, and a controllable valve can be used to direct fluid samples to the sample receiving regions separately. In this manner, a series of separate fluid samples can be obtained, optionally at different times, enabling multiple different colorimetric analyses to be performed via the sample analyzer 100.

In some embodiments, an indicator 412a is positioned inside the reservoir 402. For instance, the indicator 412a can be positioned at a distal region of the reservoir. The sample analyzer 100 can be configured to determine the analyte concentration in a fluid sample from within the reservoir 402. The analyte concentration in the fluid sample can then be communicated by the indicator 412a via display of the color of the fluid sample after reacting the fluid sample with an analytical reagent. As described herein, the analyte concentration in the fluid can be determined from the color.

Alternatively, or in combination, an indicator 412b is disposed within the cap 408. The cap 408 may be transparent, and the indicator 412b can be visible through the cap 408. In some embodiments, the sample analyzer 100 is similarly housed within the cap 408 and the sample analyzer 100 can be configured to provide an indication of the analyte concentration of a fluid sample from within the cap 408. In some examples, the cap 408 can include a sample receiving chamber having an analytical reagent, and the sample receiving chamber can be configured to react the fluid sample with the analytical reagent. The indicator 412b can be activated during a priming step to initiate the infusion set.

Alternatively, or in combination, an indicator 412c is disposed within the conduit 404. The indicator 412c can be positioned in several places in the conduit 404. For instance, the indicator 412c can be positioned in the conduit 404 distal to the cap 408. In some embodiments, the indicator 412c is positioned in the conduit 404 proximate to the cannula housing 408. An indicator 412 can also be placed on the cannula housing. The indicator 412c can be activated during a priming step to initiate the infusion set.

An imaging device, such as the imaging device 106 of the sample analyzer 100 can be utilized to determine the color of the indicators 412. For example, a separate imaging device (e.g., a smartphone, tablet, portable computing device, etc.) may be used to determine the color of the indicators 412. For example, a user can use the imaging device (e.g., smartphone) to scan the indicator 412 to get an indication of the analyte concentration. Additionally, or alternatively, an imaging device 106 can be integrated into the infusion set 400, for instance with an integrated optical detector positioned proximate to the indicator(s) 412. Signal processing components, such as signal processing components 108 of the sample analyzer 100 can be utilized to determine the analyte concentration in the fluid sample from the determined color. Optionally, the sample analyzer 100 of the infusion set 400 can be operable linked with an external device, such as a smartphone, and the indication is provided thereon.

### B. Patch Pump

Figure 5A depicts another example medicament delivery device in the form of a patch pump 500, which is designed to be worn directly on the user's skin. For clarity, the patch pump 500 is depicted with an outer shell removed to better illustrate the internal components. The patch pump 500 is a compact, self-contained unit that includes a reservoir 502 for storing the medicament and an inserter mechanism 504 that houses an internal cannula (not shown) for delivering the medicament into the patient's subcutaneous tissue. The patch pump 500 also includes a pump mechanism 506 for driving fluid from the reservoir 502 and additional electronic components 508 for controlling operation of the pump 500. A fluid path 510 extends between the reservoir 502 and the cannula (not shown) within the inserter mechanism 504, and is configured to carry fluid from the reservoir 502 to the internal cannula for subcutaneous delivery to the patient.

The reservoir 502 can hold the medicament supply for an extended period (e.g., up to 5 days or more). The reservoir 502 is usually made of a biocompatible material that can accommodate the medicament volume and withstand the pressure generated by the pump's delivery mechanism. The sample analyzer 100 can be integrated into or adjacent the reservoir 502 to monitor an analyte concentration, ensuring that the correct amount of medicament is dispensed throughout the life of the patch pump 500. For instance, the sample receiving region 104 of the sample analyzer 100 can be a sample receiving chamber 512. The sample receiving chamber 512 may include indicators 514a and 514b ("indicators 514"), as shown in Figure 5B. In some examples, each of the indicators 514 can be associated with a particular analyte concentration and the indicator 514 associated with the determined analyte concentration can be activated while other indicators 514 remain inactive. Alternatively, the indicators 514 may directly show the color of a reacted sample. In such examples, a separate imaging device (e.g., a smartphone, tablet, portable computing device, etc.) may be used to determine the color of the indicators 514, in accordance with embodiments of the present technology.

The inserter mechanism 504 is responsible for automatically inserting the internal cannula into the patient's skin and establishing a fluid path between the reservoir 502 and the subcutaneous tissue. The inserter mechanism 504 typically includes a spring-loaded needle or blade that punctures the skin and guides the cannula into place. Once the cannula is inserted, the needle or blade is automatically retracted, leaving the soft cannula in the subcutaneous tissue to deliver the medicament. The sample analyzer 100 can also be incorporated into the inserter mechanism 504, near the cannula, to measure an analyte concentration just before the fluid enters the patient's body, or during a priming operation.

In various examples, the sample analyzer 100 can be incorporated into or adjacent the fluid path 510 between the reservoir 502 and the internal cannula (not shown) within the inserter mechanism 504. Such an arrangement can beneficially enable determination of an analyte concentration within the fluid when the fluid path 510 is filled or primed, which is often performed before the pump 500 is attached to the patient. In this manner, an alert, notification, disabling of the pump 500, or other suitable action can be performed before the pump 500 is attached to the patient.

As noted above, the sample analyzer 100 can include a sample receiving region with an analytical reagent (e.g., dithizone) to facilitate colorimetric analysis of the analyte concentration in the fluid. To avoid contamination of fluid to be delivered to the patient with the analytical reagent, a sample of the fluid can be diverted to the sample receiving region in a one-way, irreversible manner. For instance, a check valve, rotatable valve, duck-bill valve, or other suitable controllable or passive one-way valve can permit a small volume of fluid sample to pass into the sample receiving region that include the analytical reagent, while not permitting the received fluid sample to exit the sample receiving region. As described previously, multiple separate sample receiving regions may be used in conjunction with controllable valves to facilitate multiple separate colorimetric analyses.

The patch pump 500 is designed to be worn continuously for a specified duration, usually 2-3 days, before being replaced with a new one. During this time, the pump's electronic components 508, including a battery, a microcontroller, and a wireless communication module, control the medicament delivery according to the programmed therapy settings. The sample analyzer 100 can provide real-time feedback to the pump's control system, enabling it to adjust the delivery rate or alert the user if any issues with the medicament concentration are detected.

### V. Conclusion

Although the devices and methods are described in the context of medicament infusion devices such as insulin pumps and pen injectors, it should be appreciated that the techniques are equally applicable to a variety of fluid delivery devices, whether other medical devices and/or non-medical devices that deliver fluid. For example, the present technology may also be applicable to devices used to deliver other medicaments such as, for example, drugs to mask pain, chemotherapy and other cancer related drugs, antibiotics, hormones, GLP-1, glucagon, various other drugs that include large molecules and proteins that may require a high level of delivery accuracy. The present technology may also be applicable to devices used to deliver non-medicament fluids.

The descriptions of embodiments of the technology are not intended to be exhaustive or to limit the technology to the precise form disclosed above. Where the context permits, singular or plural terms may also include the plural or singular term, respectively. Although specific embodiments of, and examples for, the technology are described above for illustrative purposes, various equivalent modifications are possible within the scope of the technology, as those skilled in the relevant art will recognize. For example, while steps are presented in a given order, alternative embodiments may perform steps in a different order. The various embodiments described herein may also be combined to provide further embodiments.

As used herein, the terms "generally," "substantially," "about," and similar terms are used as terms of approximation and not as terms of degree, and are intended to account for the inherent variations in measured or calculated values that would be recognized by those of ordinary skill in the art.

Moreover, within the detailed description, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

Further disclosed herein is the subject-matter of the following clauses:
1. A medicament delivery device comprising:
   a fluid path comprising a reservoir in fluid communication with an outlet port, wherein the device is actuatable to drive fluid through the fluid path, from the reservoir and out of the outlet port;
   a sample receiving chamber coupled with the fluid path, the sample receiving chamber configured to receive a fluid sample from the fluid path, wherein the sample receiving chamber comprises an analytical reagent configured to react with the fluid sample to modify a color of the fluid sample; and
   a sensor assembly comprising:
      a detector configured to determine the color of the reacted fluid sample; and
      one or more signal processing components configured to provide an indication of the analyte concentration in the fluid sample based at least in part on the color determination.
2. The device of clause 1, wherein the analyte comprises insulin.
3. The device of clause 1 or 2, wherein the analytical reagent comprises a chelating agent.
4. The device of clause 1 or of one of clauses 1 to 3, wherein the analytical reagent is configured to react with the fluid sample to form one or more coordination complexes, and the one or more coordination complexes provides the modified color of the fluid sample.
5. The device of clause 1 or of one of clauses 1 to 4, wherein the sample receiving chamber is coupled with the fluid path via a one-way valve to prevent fluid flow from the sample receiving chamber to the fluid path.
6. The device of clause 1 or of one of clauses 1 to 5, wherein the sample receiving chamber is coupled to the reservoir.
7. The device of clause 1 or of one of clauses 1 to 6, wherein the sample receiving chamber is coupled to the outlet port.
8. The device of clause 1 or of one of clauses 1 to 7, wherein the sample receiving chamber comprises one or more optical indicators, and the indication of the analyte concentration is provided via the one or more optical indicators.
9. The device of clause 1 or of one of clauses 1 to 8, wherein the signal processing components are further configured to:
   based at least in part on the indication of the analyte concentration in the fluid sample, cause an alarm to be output to a user.
10. The device of clause 1 or of one of clauses 1 to 9, wherein the signal processing components are further configured to:
   based at least in part on the indication of the analyte concentration in the fluid sample, cause a dispensation mechanism to be adjusted, wherein the adjustment varies an amount or rate of fluid dispensed via the medicament delivery device.
11. The device of clause 1 or of one of clauses 1 to 10, wherein the signal processing components are further configured to:
   based at least in part on the indication of the analyte concentration in the fluid sample, inhibit dispensation of fluid via the medicament delivery device.
12. A colorimetric analysis device comprising:
   a housing having a window;
   a sample receiving region disposed in the housing, the sample receiving region comprising an analytical reagent, wherein the sample receiving region is configured to:
      receive a fluid sample of a medicament; and
      modify a color of the fluid sample by reacting the fluid sample with the analytical reagent,
      wherein the color-modified fluid sample is visible through the window, and wherein the modified color is indicative of the analyte concentration in the fluid sample.
13. The device of clause 12, wherein the analytical reagent is configured to react with the fluid sample to form one or more coordination complexes, and the color-modified fluid sample comprises the one or more coordination complexes.
14. The device of clause 12 or 13, wherein the analyte concentration in the fluid sample can be determined from an image captured by a camera.
15. The device of clause 12 or of one of clauses 12 to 14, wherein the sample receiving region comprises a plurality of capillaries, and wherein the capillaries are configured to independently process the fluid sample.
16. The device of clause 12 or of one of clauses 12 to 15, further comprising a reference sample for calibration, wherein the reference sample contains fluid having a known color.
17. A method comprising:
   disposing a fluid sample comprising medicament within a sample receiving region, wherein the sample receiving region comprises an analytical reagent;
   reacting the fluid sample with the analytical reagent to modify a color of the fluid sample;
   determining the color of the reacted fluid sample; and
   based on the determined color, providing an indication of an analyte concentration in the fluid sample.
18. The method of clause 17, wherein the analyte comprises insulin, and wherein the analytical reagent comprises a chelating agent.
19. The method of clause 17 or 18, wherein reacting the fluid sample with the analytical reagent comprises forming one or more coordination complexes, and wherein the one or more coordination complexes produces the modified color.
20. The method of clause 17 or of one of clauses 17 to 19, wherein the sample receiving region is enclosed within a medicament delivery device configured to deliver the medicament to a patient.

## Claims

1. A medicament delivery device comprising:
a fluid path comprising a reservoir in fluid communication with an outlet port, the device being actuatable to drive fluid through the fluid path, from the reservoir and out of the outlet port;
a sample receiving chamber coupled with the fluid path, the sample receiving chamber configured to receive a fluid sample from the fluid path and having an analytical reagent configured to react with the fluid sample to modify a color of the fluid sample; and
a sensor assembly comprising:
a detector configured to determine the color of the reacted fluid sample; and
one or more signal processing components configured to provide an indication of the analyte concentration in the fluid sample based at least in part on the color determination.

2. The device of claim 1, wherein the analyte comprises insulin.

3. The device of claim 1 or 2, wherein the analytical reagent comprises a chelating agent.

4. The device of claim 3, wherein the chelating agent is dithizone.

5. The device of any one of the preceding claims, wherein the analytical reagent is configured to react with the fluid sample to form one or more coordination complexes, and the one or more coordination complexes provides the modified color of the fluid sample.

6. The device of claim 5, wherein the detector is configured to determine the color of the reacted fluid sample by determining a dominant wavelength of the one or more coordination complexes.

7. The device of any one of the preceding claims, wherein the sample receiving chamber is coupled with the fluid path via a one-way valve to prevent fluid flow from the sample receiving chamber to the fluid path.

8. The device of any one of the preceding claims, wherein the sample receiving chamber is coupled to the reservoir.

9. The device of any one of the preceding claims, wherein the sample receiving chamber is coupled to the outlet port.

10. The device of any one of the preceding claims, wherein the sample receiving chamber is releasably coupled with the fluid path.

11. The device of any one of the preceding claims, wherein the sample receiving chamber comprises one or more optical indicators, and the indication of the analyte concentration is provided via the one or more optical indicators.

12. The device of any one of the preceding claims, wherein the signal processing components are further configured to:
based at least in part on the indication of the analyte concentration in the fluid sample, cause an alarm to be output to a user.

13. The device of any one of the preceding claims, wherein the signal processing components are further configured to:
based at least in part on the indication of the analyte concentration in the fluid sample, cause a dispensation mechanism to be adjusted, wherein the adjustment varies an amount or rate of fluid dispensed via the medicament delivery device.

14. The device of any of the preceding claims, wherein the signal processing components are further configured to:
based at least in part on the indication of the analyte concentration in the fluid sample, inhibit dispensation of fluid via the medicament delivery device.
